# EUROPÄISCHE PATENTSCHRIFT

(11) **EP 2 833 936 B1**
(45) Veröffentlichungstag und Bekanntmachung des Hinweises auf die Patenterteilung: **01.06.2016**
(21) Anmeldenummer: 13712127.3
(22) Anmeldetag: 14.03.2013
(51) Int. Cl.: A61M 1/00, A61M 1/06, A01J 5/04

(54) **SAUGPUMPENEINHEIT**
SUCTION PUMP UNIT
ENSEMBLE DE POMPE ASPIRANTE

(30) Priorität: 04.04.2012 CH 474122012
(43) Veröffentlichungstag der Anmeldung: 11.02.2015
(73) Patentinhaber: Medela Holding AG, 6340 Baar (CH)
(72) Erfinder: FELBER, Armin, CH-6003 Luzern (CH)
(74) Vertreter: Clerc, Natalia
(86) Internationale Anmeldenummer: PCT/CH2013/000040
(87) Internationale Veröffentlichungsnummer: WO 2013/149351

(56) Entgegenhaltungen:
- DE-A1- 2 347 034
- DE-A1- 10 228 455
- US-A- 4 070 856
- US-A- 4 487 224
- US-A- 4 607 596
- US-A1- 2005 283 112
- US-A1- 2010 121 265
- US-A1- 2010 324 473

## Beschreibung

### TECHNISCHES GEBIET

Die vorliegende Erfindung betrifft eine Saugpumpeneinheit gemäss Oberbegriff des Patentanspruchs 1 sowie ein Verfahren zum Betreiben einer Saugpumpeneinheit gemäss Oberbegriff des Patentanspruchs 18. Die Saugpumpeneinheit dient insbesondere zur Verwendung als Brustpumpe zum Absaugen von menschlicher Muttermilch.

### STAND DER TECHNIK.

Saugpumpeneinheiten sind im Stand der Technik hinlänglich bekannt. Sie werden zum Absaugen von Muttermilch, aber auch im medizinischen Bereich zum Absaugen von Körperflüssigkeiten bzw. - fluiden eingesetzt. Beispiele hierfür sind Absaugprozesse während und nach chirurgischen Eingriffen, die Wunddrainage, die Thoraxdrainage und das Absaugen von Körperfetten.

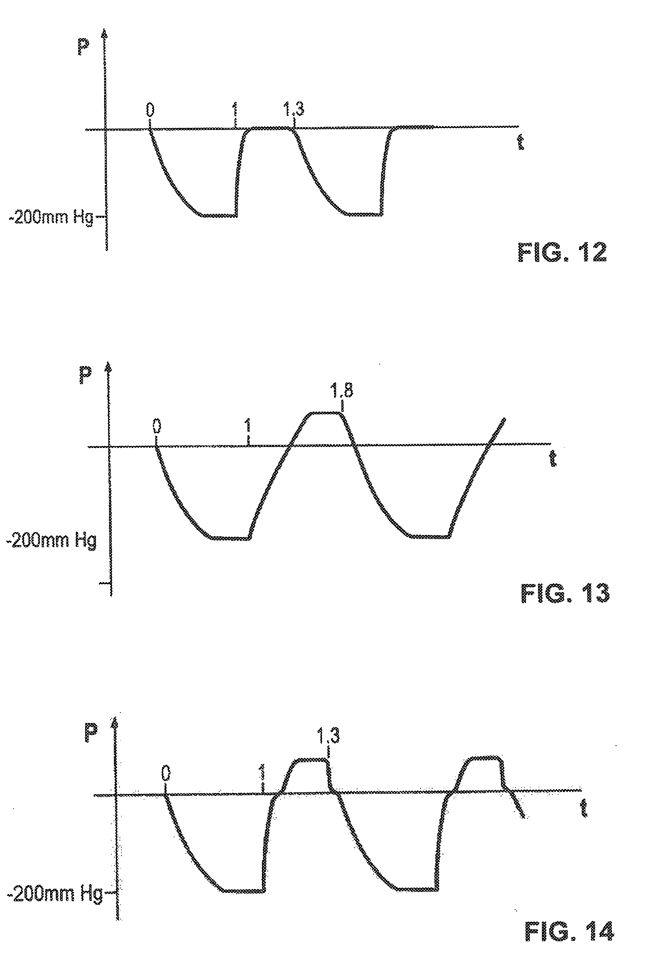

Beim Abpumpen von Muttermilch wird mit Hilfe einer Vakuumpumpe ein Unterdruck in einer an die Mutterbrust angelegten Brusthaube geschaffen, wodurch die Muttermilch aus der Brust abgesaugt wird. Um ein möglichst schmerzfreies und dem Saugrhythmus des Säuglings entsprechendes Saugen zu ermöglichen, wird ein sich zeitlich verändernder Unterdruck an die Brusthaube angelegt. Saugkurven, welche einen schnellen Anstieg des Unterdrucks auf Atmosphärendruck oder auf einen anderen Basiswert aufweisen, haben sich in der Praxis besonders bewährt. Ein entsprechender Druckverlauf ist in Figur 12 dargestellt.

Es hat sich gezeigt, dass ein Anstieg des Drucks in einen Überdruckbereich vorteilhaft sein kann, wie dies in Figur 13 erkennbar ist. Ein Vorteil ist beispielsweise, dass Milch, welche in den Vakuumschlauch zwischen Brusthaube und Brustpumpe gelangt ist, ausgeblasen werden kann. Ein weiterer Vorteil ist, dass sich bei aktiver Entlüftung dank Überdruckquelle ein relativ dünner Vakuumschlauch zwischen Brusthaube und Pumpaggregat verwenden lässt. Ohne aktive Entlüftung wäre der Strömungswiderstand im dünnen Schlauch zu gross, so dass das Vakuum nicht genügend schnell abgebaut werden könnte. Dünne Schläuche bilden ein kleines Totvolumen. Dadurch lassen sich wiederum kleinere Pumpaggregate, d.h. Vakuumpumpen mit kleinerer Saugleistung, einsetzen.

Ein Druckverlauf mit positivem Anteil kann erreicht werden, indem der Auspuff der Vakuumpumpe als Druckquelle verwendet wird. Um die Funktionsweise der Vakuumpumpe zum gewährleisten, muss jedoch stets einer der Abschlüsse der Pumpe, d.h. der Vakuumanschluss oder der Auspuff, offen sein. Dies lässt sich mit einem 5/2 Wegventil bewerkstelligen. Diese Ventile haben jedoch den Nachteil, dass sie relativ gross, schwer und teuer sind. Zudem ist auch bei diesem Druckverlauf eine steile Flanke beim Anstieg des Drucks bevorzugt. Eine derartig steile Flanke lässt sich jedoch kaum mit 5/2 Wegventilen erzielen.

DE 102 28 455 offenbart eine Milchabsaugvorrichtung mit einer Vakuumpumpe, deren Vakuumanschluss und deren Auspuff mit einem Umschaltventil verbunden sind. Vom Umschaltventil führt eine Saugleitung zu einer Brusthaube. Dank der Verbindung des Umschaltventils mit dem als Überdruckquelle agierenden Auspuff lässt sich der Unterdruck in der Brusthaube relativ schnell ändern. Das Umschaltventil weist in diesem Fall eine rotierende Scheibe auf, welche abwechslungsweise eine Unterdrucköffnung oder eine Überdrucköffnung freigibt.

US 2010/0121265 beschreibt eine Brustpumpe zum Abpumpen von Muttermilch mit Solenoidventilen.

Die mit bekannten Saugpumpeneinheiten erzielbaren Druckänderungen mit Hilfe einer Überdruckquelle erfolgen relativ langsam. Um ein möglichst naturgetreues Abpumpen zu ermöglichen, sind jedoch schnelle Druckänderungen notwendig. Auch in den übrigen oben erwähnten Anwendungsgebieten von medizinalen Saugpumpeneinheiten besteht das Bedürfnis, Saugkurven mit sich rasch ändernden Druckverhältnissen anzubieten.

### DARSTELLUNG DER ERFINDUNG

Es ist deshalb eine Aufgabe der Erfindung, eine Saugpumpeneinheit und ein Verfahren zum Betreiben einer derartigen Saugpumpeneinheit zu schaffen, welche eine schnelle und sichere Reduktion des applizierten Unterdrucks auch bei Verwendung einer Überdruckquelle ermöglicht.

Diese Aufgabe lösen eine Saugpumpeneinheit mit den Merkmalen des Patentanspruchs 1 und ein Verfahren zum Betreiben einer Saugpumpeneinheit mit den Merkmalen des Patentanspruchs 18.

Die erfindungsgemässe Saugpumpeneinheit weist einen Unterdruckanschluss, einen Überdruckanschluss und ein Umschaltventil auf. Das Umschaltventil weist einen Ventilkörper mit zwei Eingängen auf, wobei ein erster der Eingänge mit dem Unterdruckanschluss und ein zweiter der Eingänge mit dem Überdruckanschluss verbunden sind. Das Umschaltventil weist ferner einen Ausgang mit einer Durchgangsöffnung auf, wobei der Ausgang relativ zu den Eingängen vom ersten Eingang zum zweiten Eingang und zurück bewegbar ist. Er ist somit zwischen diesen zwei Eingängen hin und her schaltbar. Die Durchgangsöffnung bildet dadurch abwechslungsweise mit einem der Eingänge eine fluidkommunizierende Verbindung. Erfindungsgemäss ist die Durchgangsöffnung des Ausgangs während mindestens einem Teil der Bewegung zwischen den zwei Eingängen freigegeben und belüftet.

In einer bevorzugten Ausführungsform ist der Ausgang beim Bewegen zwischen den zwei Eingängen relativ zu den zwei Eingängen anhebbar, wobei die Durchgangsöffnung des Ausgangs im angehobenen Zustand freigegeben und belüftet ist.

Im erfindungsgemässen Verfahren zum Betreiben einer oben genannten Saugpumpeneinheit wird der Ausgang relativ zu den zwei Eingängen zwischen den zwei Eingängen hin und her bewegt. Die Durchgangsöffnung erstellt beim Bewegen des Ausgangs abwechslungsweise mit einem der Eingänge eine fluidkommunizierende Verbindung. Erfindungsgemäss wird die Durchgangsöffnung des Ausgangs beim Bewegen zwischen den zwei Eingängen freigegeben und belüftet.

Erfindungsgemäss werden der Ausgang und eine mit diesem Ausgang verbundene Ausgangsleitung bereits belüftet, sobald der Ausgang vom Unterdruckanschluss entfernt wird und bevor er mit dem Überdruckanschluss verbunden ist. Die Belüftung erfolgt dadurch schneller als bei herkömmlichen 5/2 Wegventilen. Ein entsprechender Druckverlauf ist in Figur 14 dargestellt. Es lassen sich beispielsweise Entlüftungszeiten und somit Druckanstieg in den Atmosphärenbereich erreichen, welche den klassischen Saugpumpeneinheiten ohne Überdruckquellen entsprechen. Darüber hinaus lässt sich jedoch auch ein Druckanstieg in den positiven Wertebereich erhalten.

Dank der steilen Druckanstiegs- bzw. Belüftungskurve lassen sich kürzere Zykluszeiten und damit höhere Zykluszahlen erreichen als bei bekannten Saugpumpeneinheiten mit positivem Druckverlauf. Die Pumpfrequenz kann dadurch erhöht werden.

Dank dieses erfindungsgemässen Umschaltventils ist die Bandbreite der zur Verfügung stehenden Saugkurven sowohl bezüglich möglicher Pumpfrequenzen, Pumpleistungsänderungen und auch bezüglich der Form der Kurve massiv vergrössert.

Wird eine Membran zur Medientrennung verwendet, wie dies beispielsweise in WO 2011/035447 beschrieben ist, so erfolgt dank der raschen Druckänderung die Rückstellung dieser Membran schnell und zuverlässig. Auch dies ermöglicht die Verwendung einer höheren Zyklenzahl der Saugkurve im Vergleich zu bekannten Saugpumpen.

Vorteilhaft ist ferner, dass das erfindungsgemässe Umschaltventil, auch Stellglied genannt, relativ klein und einfach aufgebaut ist. Es ist entsprechend kostengünstig. Vorteilhaft ist ferner, dass es lediglich ein Dreiwegeventil ist.

Der Ausgang wird relativ zu den Eingängen bewegt, das heisst es kann entweder der Ausgang und/oder die Eingänge bewegt werden. In einer bevorzugten Ausführungsform ist der Ventilkörper mit den Eingängen lagefixiert in der Saugpumpeneinheit angeordnet und der Ausgang wird bewegt.

Vorzugsweise ist die relative Bewegung des Ausgangs zu den zwei Eingängen eine Kombination einer geradlinigen Verschiebung in einer ersten Richtung und einer Anhebung in einer zur ersten Richtung senkrechten zweiten Richtung. Diese Bewegung ermöglicht ein frühzeitiges Freigeben der Durchgangsöffnung des Ausgangs, wobei bereits eine minimale Kraft zum Lösen des Ausgangs vom entsprechenden Eingang ausreicht.

Vorzugsweise ist am Ausgang eine Ausgangsleitung der Saugpumpeneinheit befestigt, welche gemeinsam mit dem Ausgang bewegbar ist. Diese Ausgangsleitung führt zum Verbraucher. Im Falle einer Brustpumpeneinheit führt sie zur Brusthaube. Im Falle einer Drainagepumpe führt sie zu einer abzusaugenden Kavität des Patienten.

Die Saugpumpeneinheit kann eine Unterdruckquelle und eine davon getrennte und unabhängige Überdruckquelle aufweisen. Vorzugsweise wird jedoch ein Auspuff einer Vakuumpumpe als Überdruckquelle verwendet. In einer bevorzugten Ausführungsform weist die Saugpumpeneinheit deshalb eine einzige Vakuumpumpe auf, wobei die Vakuumpumpe den Unterdruckanschluss aufweist und wobei sie ferner einen Auspuff aufweist, welcher den Überdruckanschluss bildet.

Um den relativen Verschiebungsweg des Ausgangs möglichst klein zu halten, sind die zwei Eingänge vorzugsweise in einer Ebene nebeneinander angeordnet.

In einer bevorzugten Ausführungsform sind die zwei Eingänge in einer Gleitplatte mit einer planen Oberfläche angeordnet, und der Ausgang liegt bei erstellter fluidkemmunizierender Verbindung mit den Eingängen auf dieser Gleitplatte auf. Diese Gleitplatte ermöglicht eine dichte Verbindung zwischen Eingang und Ausgang. Wenn die Gleitplatte einen niedrigen Haft- und Gleitreibungskoeffizienten aufweist, lässt sich der Ausgang mit geringem Kraftaufwand relativ zu den Eingängen bewegen.

Die Relativbewegung des Ausgangs in Bezug auf die zwei Eingänge lässt sich auf unterschiedliche Weise erzielen. In einer bevorzugten Ausführungsform ist das erfindungsgemässe Umschaltventil ein Elektromagnetventil.

In einer bevorzugten Ausführungsform weist es zwei Elektromagnete mit je einer Spule auf, wobei jede Spule von einem ferromagnetischen hohlen Kern durchsetzt ist. Die zwei hohlen Kerne bilden die zwei Eingänge. Der Ausgang weist einen Permanentmagneten auf. Vorzugsweise ist der Permanentmagnet ein Ring, welcher die Durchgangsöffnung bildet. In einem bevorzugten Ausführungsbeispiel ist der Ausgang durch einen Permanentmagneten, insbesondere durch die ringförmige Ausführungsform, gebildet.

Die Spulen sind vorzugsweise parallel zueinander verlaufend angeordnet. Sie können jedoch auch in einem Winkel zueinander angeordnet sein, wobei sie sich zum Ausgang hin annähern. Dadurch ist der Weg, auf welchem sich der Ausgang zwischen den zwei Eingängen hin- und her bewegt, im Vergleich zur parallelen Anordnung verkürzt. Die Umschaltzeit ist dadurch minimiert.

Die Verwendung von elektromagnetischen Spulen, insbesondere von Zylinderspulen, im Umschaltventil ermöglicht ein sehr schnelles Umschalten des Ventils. Die zwei Spulen werden mit entgegen gesetztem Strom beaufschlagt. Bei Richtungsänderung der zwei Ströme schaltet das Ventil um. Diese Ausführungsform weist den weiteren Vorteil auf, dass sie kaum Materialermüdungen unterliegt und ein sicheres Umschalten des Ventils gewährleistet.

Wird ein Permanentmagnet im Ausgang verwendet, so wird der Ausgang im stromlosen Zustand der Spulen von einem der Kerne angezogen und schliesst das Ventil in dieser Stellung. Die pneumatische Dichtheit ist auch in diesem Zustand gewährleistet. Das Umschaltventil weist einen geringen Stromverbrauch auf, da die Spulen nur beim Wechsel von Unterdruck zu positivem Druck und umgekehrt Strom benötigen.

Damit der Ausgang in seiner Relativbewegung in Bezug auf die zwei Eingänge genügend geführt ist, sind in einer bevorzugten Ausführungsform die zwei Eingänge des Ventilkörpers von einem Deckel überdeckt. Der Deckel lässt über den Eingängen einen Hohlraum frei. Der Ausgang ist in diesem Hohlraum bewegbar gehalten. Im Hohlraum ist Luft für die Belüftung des Ausgangs während seiner Hin- und Herbewegung vorhanden. Vorzugsweise ist der Hohlraum hierfür mit Umgebungsluft beaufschlagt, d.h. das Gehäuse des Ventils ist gegenüber der Umgebung nicht dicht oder es weist eine Luftzufuhröffnung auf. Unter Umgebung wird hier entweder das Innere der Saugpumpeneinheit oder die äussere Umgebung der Saugpumpeneinheit verstanden.

Damit der Anschluss in seiner Hin- und Herbewegung relativ zu den Eingängen angehoben werden kann, weist der Hohlraum vorzugsweise eine Höhe auf, welche grösser als eine Dicke des Anschlusses ist. Andere Lösungen, wie beispielsweise eine Absenkbewegung der Eingänge, sind möglich.

Um die Stabilität des Ventils zu gewährleisten, sind in einer bevorzugten Ausführungsform die zwei Spulen in einem gemeinsamen Joch gehalten, wobei das Joch an einem dem Ausgang gegenüberliegenden Ende des Ventilkörpers angeordnet ist.

Anstelle von elektromagnetischen Spulen kann das Umschaltventil auch als Parallelogramm ausgebildet sein. In einer bevorzugten Ausführungsform weist das Umschaltventil zwei parallel zueinander angeordnete und parallel zueinander verschiebbare Platten auf., wobei in einer ersten dieser Platten der Ausgang gehalten ist und in einer zweiten dieser Platten die zwei Eingänge angeordnet sind. Vorzugsweise sind die zwei Platten mittels Biegeelementen, insbesondere Blattfedern oder Flächenelementen mit Filmgelenken, miteinander zu einem Parallelogramm verbunden.

Vorzugsweise erfolgt die Relativbewegung zwischen dem Ausgang und den Eingängen dadurch, dass mindestens eine dieser zwei Platten durch Kraftbeaufschlagung in Richtung ihrer Plattenebene bewegt wird. Vorzugsweise sind die Eingänge fest gehalten und der Ausgang wird bewegt.

Die Ausführungsformen mit dem Parallelogramm weisen den Vorteil auf, dass sie leichter und kostengünstiger sind als die Varianten mit den elektromagnetischen Spulen.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung einer erfindungsgemässen Brustpumpeneinheit während des Abpumpens von Muttermilch;
- Figur 2: eine perspektivische Darstellung eines erfindungsgemässen Umschaltventils in einer ersten Ausführungsform;
- Figur 3: einen Längsschnitt durch das Umschaltventil gemäss Figur 2;
- Figur 4: eine Ansicht von oben des Umschaltventils gemäss Figur 2;
- Figur 5: eine weitere perspektivische Darstellung des Umschaltventils gemäss Figur 2;
- Figur 6: einen Längsschnitt durch das Umschaltventil gemäss Figur 2 in einer ersten Position des Ausgangs;
- Figur 7: einen Längsschnitt durch das Umschaltventil gemäss Figur 2 in einer zweiten Position des Ausgangs;
- Figur 8: einen Längsschnitt durch das erfindungsgemässe Umschaltventil in einer zweiten Ausführungsform und in einer ersten Position;
- Figur 9: einen Längsschnitt durch das Umschaltventil gemäss Figur 8 in einer zweiten Position;
- Figur 10: eine schematische Darstellung der Hin- und Herbewegung des Ausgangs des Umschaltventils gemäss Figur 8;
- Figur 11: einen vergrösserten Ausschnitt eines erfindungsgemässen Umschaltventils in einer dritten Ausführungsform;
- Figur 12: eine graphische Darstellung des Drucks in Funktion der Zeit einer Brustpumpe gemäss dem Stand der Technik ohne positiven Druck;
- Figur 13: eine graphische Darstellung des Drucks in Funktion der Zeit einer Brustpumpe gemäss dem Stand der Technik mit positiven Druck und
- Figur 14: eine graphische Darstellung des Drucks in Funktion der Zeit einer erfindungsgemässen Brustpumpe.

### BESCHREIBUNG BEVORZUGTER AUSFÜHRUNGSFORMEN

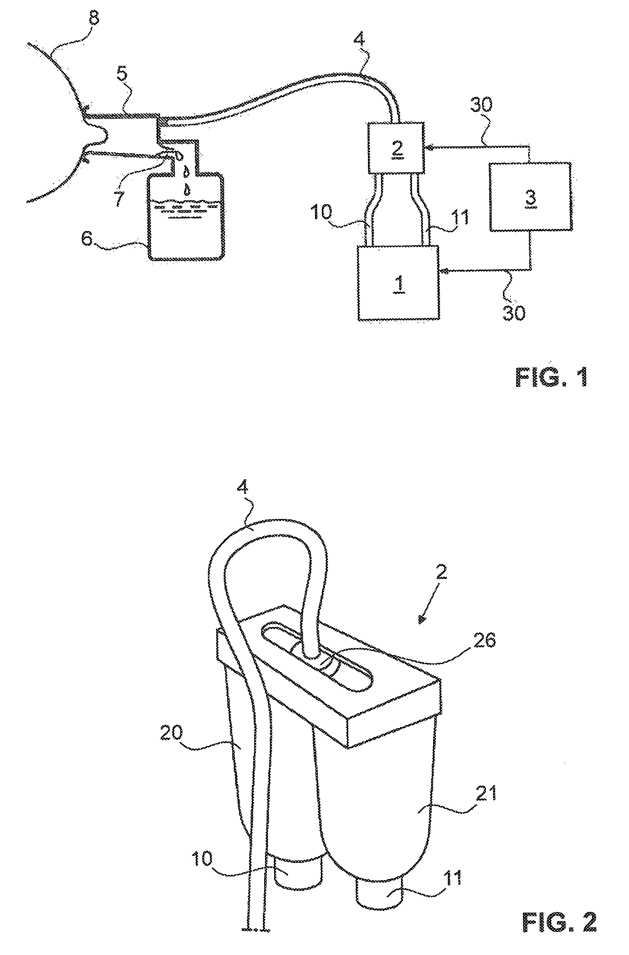

In Figur 1 ist eine schematische Brustpumpeneinheit dargestellt. Die erfindwigsgemässe Lehre lässt sich jedoch auch auf andere Saugpumpen, insbesondere auf Drainagepumpen, übertragen. Die Saugpumpeneinheit weist eine Saugpumpe 1, auch Vakuumaggregat genannt, auf. Die Saugpumpe 1 weist einen Saug- oder Vakuumanschluss und einen Auspuff zur Entlüftung der Saugpumpe 1 auf. Am Sauganschiuss ist eine Saugleitung 10 angeschlossen, am Auspuff eine Druckleitung 11. Diese zwei Leitungen 10, 11 führen zu einem Umschaltventil 2, auch Stellglied genannt. Die Saugpumpe 1 und das Umschaltventil 2 sind über eine elektronische Steuerung 3 miteinander verbunden. Die Verbindungsleitungen sind schematisch dargestellt und mit dem Bezugszeichen 30 versehen.

Vom Umschaltventil 2 führt eine Ausgangsleitung 4 zu einer Brusthaube 5, welche mit einem Milchsammelbehälter 6 verbunden ist. Zwischen Milchsammelbehälter 6 und Brusthaube 5 ist üblicherweise ein Rückschlagventil 7 angeordnet, welches das Totvolumen begrenzt. Die menschliche Mutterbrust, auf welche die Brusthaube 5 aufgesetzt ist, ist hier mit dem Bezugszeichen 8 versehen. Die Brusthaube 5 kann auch eine andere Form aufweisen. Beispiele hierfür sind aus dem Stand der Technik hinlänglich bekannt. Die Ausgangsleitung 4 ist üblicherweise ein flexibler Schlauch, insbesondere aus Silikon. Die Saugpumpe 1 mit dem Umschaltventil 2 kann jedoch auch direkt an der Brusthaube 5 befestigt sein, so dass die Ausgangsleitung 4 entfällt bzw. innerhalb eines Gehäuses einer Brusthauben-Saugpumpenkombination verläuft.

In den Figuren 2 bis 7 ist ein erstes Ausführungsbeispiel eines erfindungsgemässen Umschaltventils 2 dargestellt. Es weist zwei Elektromagnete, d.h. Zylinderspulen 20, 21 auf. In diesen Spulen 20, 21 verlaufen ferromagnetische Kerne 22, 23, welche ebenfalls vorzugsweise als Hohlzylinder ausgebildet sind. Als Kerne 22, 23 eignen sich insbesondere Strahl- oder Eisenkerne. Im Folgenden wird deshalb von Eisenkernen gesprochen, wobei die Kerne auch aus anderen geeigneten Materialien bestehen können. Diese Kerne 22, 23 bilden zwei Eingänge des Umschaltventils 2. Ein erster Kern 22 der ersten Spule 20 ist mit der Saugleitung 10, ein zweiter Kern 23 der zweiten Spule 21 mit der Druckleitung 11 der Saugpumpe 1 verbunden.

Die zwei Spulen 20, 21 sind in einem Joch 27 gehalten. Das Joch 27 besteht vorzugsweise ebenfalls aus einem ferromagnetischen Material. Die zwei Kerne 22, 23 berühren oder durchsetzen das Joch 27 vorzugsweise.

Am gegenüberliegenden Ende sind die zwei Spulen 20, 21 von einer Gleitplatte 24 überdeckt. Die zwei Kerne 22, 23 ragen vorzugsweise in diese Platte 24 hinein. Gleitplatte 24, Spulen 20, 21 und Kerne 22, 23 bilden einen Ventilkörper.

Die Platte 24 weist Durchgangsöffnungen 240, 241 auf, wobei eine erste dieser Durchgangsöffnungen 240 mit dem durch den ersten Kein 22 gebildeten ersten Eingang verbunden ist. Eine zweite der Durchgangsöffnungen 241 ist mit dem durch den zweiten Kern 23 gebildeten zweiten Eingang verbunden. Dadurch ist eine erste fluiddurchlässige Leitung vom Sauganschluss über die Saugleitung 10 zum ersten Eingang 22 und zur ersten Durchgangsöffnung 240 geschaffen. Eine zweite fluiddurchlässige Leitung ist vom Auspuff über die Druckleitung 11 zum zweiten Eingang 23 und zur zweiten Durchgangsöffnung 241 geschaffen. Vorzugsweise weisen beide Durchgangsöffnungen 240, 241 denselben Durchmesser auf. Auch die zwei Eingänge 22,23 weisen vorzugsweise denselben Durchmesser auf. Die Durchmesser der Durchgangsöffnungen 240, 241 sind gleich gross wie die Durchmesser der Eingänge 22, 23 oder vorzugsweise sind sie kleiner. Sie sind jedoch vorzugsweise zentrisch auf den Eingängen 22, 23 angeordnet.

Die Gleitplatte 24 besteht vorzugsweise aus Kunststoff, insbesondere aus Polyamid. Sie weist vorzugsweise eine nach aussen gerichtete plane Oberfläche auf. Diese kann zumindest im Bereich der Kerne 22, 23 einen geringen Haft- und Gleitreibungskoeffizienten aufweisen.

Diese Gleitplatte 24 ist von einem Deckel 25 überdeckt. Der Deckel ist vorzugsweise aus Kunststoff gefertigt, insbesondere aus Polyamid. Er bildet einen Hohlraum 250 über der Gleitplatte 24, welche ein Fenster, hier einen Schlitz 251 aufweist. Das Fenster 251 erstreckt sich mindestens von der ersten Durchgangsöffnung 240 zur zweiten Durchgangsöffnung 241. Das Fenster ist von einer nach innen gerichteten Oberfläche 252 des Deckels 25 umgeben. Diese Oberfläche 252 weist vorzugsweise einen geringen Haft- und Gleitreibungskoeffizienten auf.

Im Hohlraum 250 ist ein Permanentmagnetring 26 angeordnet. Er weist eine Durchgangsöffnung auf, welche annähernd den Durchgangsöffnungen 240, 241 der Gleitplatte 24 entspricht. Der Permanentmagnetring 26 ist verschiebbar im Hohlraum 250 gehalten, wobei der Hohlraum 250 so bemessen ist, dass sich der Ring 26 von der ersten Durchgangsöffnung 240 und somit vom ersten Eingang 22 zur zweiten Durchgangsöffnung 241 und somit zum zweiten Eingang 23 hin und zurück bewegen lässt. Dabei überdeckt der Magnetring 26 die jeweilige Durchgangsöffnung 240, 241 vollständig und bildet eine fluiddichte Verbindung mit dem jeweiligen Eingang 22, 23.

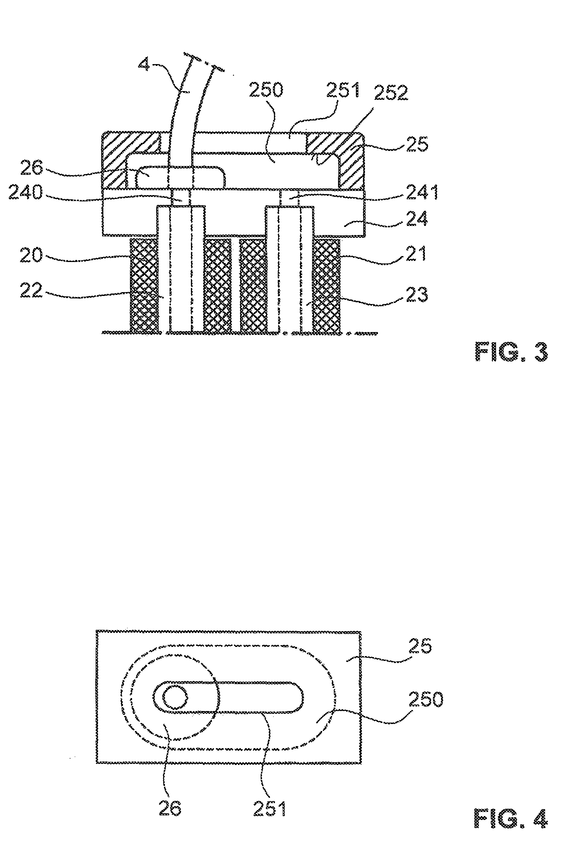

Der Magnetring 26 bildet den Ausgang des Umschaltventils 2. Am Magnetring ist die Ausgangsleitung 4 angeschlossen. Der Schlitz 251 ist genügend gross, um die Ausgangsleitung 4 ungehindert bewegen zu lassen, wenn sich der Magnetring 26 bewegt. Dies ist in den Figuren 3 und 4 gut erkennbar. Die Ausgangsleitung 4 ist vorzugsweise u-förmig vom Deckel 25 weggeführt, wie dies in Figur 2 erkennbar ist.

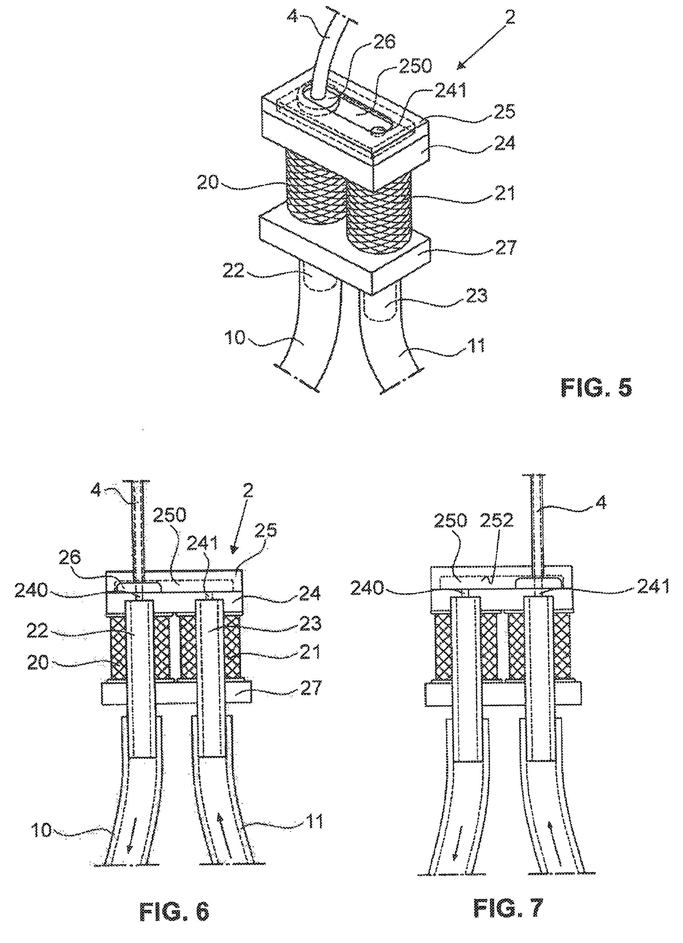

Durch die beschriebene Anordnung lässt sich wahlweise eine dichte Leitung zwischen Sauganschluss der Vakuumpumpe 1 und Brusthaube 5 und eine dichte Leitung zwischen Auspuff der Vakuumpumpe 1 und Brusthaube 5 erstellen. Hierfür werden die Spulen 20, 21 kurzzeitig mit gegenläufigem Strom beaufschlagt. Das dadurch im Kern 22 erzeugte Magnetfeld ist umgekehrt zum Magnetfeld im zweiten Kern 23 gepolt. Der Magnetring 26 wird von einem Kern 22 angezogen und vom anderen Kern 23 abgestossen. Diese Situation ist in den Figuren 5 und 6 dargestellt. Die Saugleitung 10 ist nun mit der Brusthaube 5 verbunden.

Wird die Stromrichtung gewechselt d.h. die Spulen umgepolt, so werden auch die Magnetfelder umgepolt. Der Magnetring 26 wird nun vom ersten Eingang 22 abgestossen und vom zweiten Eingang 23 angezogen. Er bewegt sich zum zweiten Eingang 23 hin, wobei er dabei zum Deckel 25 hin angehoben wird und somit seine Durchgangsöffnung freigegeben wird. Die Anhebung des Magnetrings 26 wird durch den Deckel 25 begrenzt. Dies ist in Figur 3 gut erkennbar. Der Magnetring 26 gleitet dabei vorzugsweise entlang der nach innen gerichteten Oberfläche 252 des Deckels 25 zum zweiten Eingang 23 hin und er senkt sich auf diesen zweiten Eingang 23 herab.

Der Magnetring, d.h. der Ausgang 26 und somit die Ausgangsleitung 4 werden durch die Anhebung des Ausgangs 26 belüftet. Am Ende seiner Bewegung liegt der Magnetring 26 auf dem zweiten Eingang 23 und bildet mit diesem eine fluiddichte Leitung. Nunmehr sind der Auspuff und die Druckleitung 11 mit der Brusthaube 5 verbunden. Die Ausgangsleitung 4 und die Brusthaube 5 können aktiv belüftet werden und es kann sogar ein Überdruck erzeugt werden. Durch erneutes Umschalten des Stroms wird der Magnetring 26 wiederum zum ersten Eingang 22 zurück bewegt. Die Bewegung des Magnetrings 26 ist dabei dieselbe wie oben beschrieben, jedoch in umgekehrter Richtung.

Fliesst kein Strom, so verbleibt der Magnetring 26 an seiner letzten Position und bildet nach wie vor eine dichte Leitung.

Die Umschaltung des Stroms und somit das Umpolen der Magnetfelder erfolgt nach Massgabe der Steuerung 3. Es sind hier die unterschiedlichsten Betätigungsmuster möglich, welche zyklisch, erratisch oder anderen Vorgaben folgend sein können.

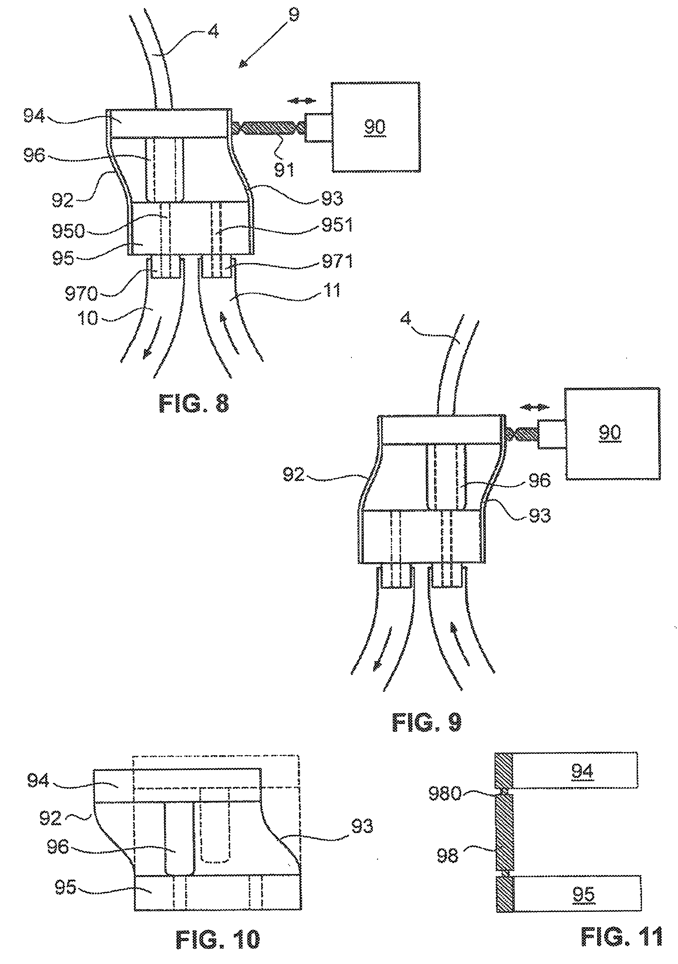

In den Figuren 8 bis 10 ist ein zweites Ausführungsbeispiel des erfindungsgemässen Umschaltventils beschrieben. Gleiche Teile sind mit gleichen Bezugszeichen wie im ersten Beispiel bezeichnet.

Anstelle von Permanentmagneten ist hier ein Parallelogramm 9 vorhanden, welches durch zwei Platten 94, 95 und zwei Biegeteile 92, 93 gebildet ist. Die zwei Platten sind vorzugsweise aus Metall oder Kunststoff gefertigt. An der ersten Platte 94 ist der Ausgang 96 des Umschaltventils angeordnet. Hier ist er mit der ersten Platte 94 fest verbunden. Der Ausgang 96 ist auch mit der Ausgangsleitung 4 verbunden. Beispielsweise durchsetzt entweder die Ausgangsleitung 4 und/oder der Ausgang 96 die erste Platte 94. In der zweiten Platte 95, welche einen Ventilkörper bildet, sind die zwei Eingänge in Form von Durchgangsbohrungen 950, 951 vorhanden. Sie sind vorzugsweise nebeneinander angeordnet. An der zweiten Platte 95 sind Anschlussstutzen 970, 971 vorhanden, um die Saugleitung 10 und die Druckleitung 11 anzuschliessen.

Der Ausgang 96 ist wiederum relativ zu den Eingängen bewegbar. Hierfür ist ein Antrieb 90 vorhanden. Dieser Antrieb kann beispielsweise ein Elektromotor sein. Der Antrieb 90 ist mit einer Antriebsstange 91 verbunden, welche mit einer seitlichen Stirnfläche der ersten Platte 94 fest verbunden ist oder an ihr anliegt. Die erste Platte 94 lässt sich somit entlang ihrer Plattenebene verschieben. Dadurch verschiebt sich auch der Ausgang 96 vom ersten Eingang 950 gemäss Figur 8 zum zweiten Eingang 951 gemäss Figur 9.

Die Bewegung ist wie im ersten Ausführungsbeispiel eine Kombination einer geradlinigen Verschiebung in einer ersten Richtung und einer Anhebung in einer zur ersten Richtung senkrechten zweiten Richtung. Dies ist in Figur 10 gut erkennbar.

Sind die Biegeteile wie in diesem Beispiel Blattfedern 92, 93, so wird die Bewegung noch zusätzlich unterstützt. Es ist auch möglich, die Antriebsstange 91 lediglich für die Bewegung in einer Richtung zu verwenden und die Rückstellung über die Blattfedern ausführen zu lassen. Des Weiteren können mehrere Antriebsstangen an verschiedenen Kraftansetzungspunkten des Parallelogramms verwendet werden. Alternativ kann zudem anstelle der ersten Platte 94 oder zusätzlich zur ersten Platte 94 die zweite Platte 95 bewegt werden.

In Figur 11 ist ein drittes Ausführungsbeispiel dargestellt. Es entspricht der Lösung gemäss den Figuren 8 bis 10, ausser dass anstelle von Blattfedern Flächenelemente 98 mit Filmscharnieren 980 verwendet werden. Diese Flächenelemente 98 können beispielsweise aus Metall oder Kunststoff gefertigt sein.

Die erfindungsgemässe Saugpumpeneinheit mit dem Umschaltventil ermöglicht ein passives und aktives Belüften der mit dem Ausgang verbundenen Ausgangsleitung.

**BEZUGSZEICHENLISTE**

| | | | |
|---|---|---|---|
| 1 | Saugpumpe | | |
| 10 | Saugleitung | 5 | Brusthaube |
| 11 | Druckleitung | | |
| | | 6 | Milchsammelbehälter |
| 2 | Umschaltventil | | |
| 20 | erste Spule | 7 | Rückschlagventil |
| 21 | zweite Spule | | |
| 22 | erster ferromagnetischer Kern | 8 | Mutterbrust |
| 23 | zweiter ferromagnetischer | | |
| | Kern | 9 | Parallelogramm |
| 24 | Gleitplatte | 90 | Antrieb |
| 240 | erste Durchgangsöffnung | 91 | Antriebsstange |
| 241 | zweite Durchgangsöffnung | 92 | erste Blattfeder |
| 25 | Deckel | 93 | zweite Blattfeder |
| 250 | Hohlraum | 94 | erste Platte |
| 251 | Schlitz | 95 | zweite Platte |
| 252 | innere Oberfläche | 950 | erste Durchgangsbohrung |
| 26 | Permanentmagnetring | 951 | zweite Durchgangsbohrung |
| 27 | Joch | 96 | Umschaltleitung |
| | | 970 | erster Anschlussstutzen |
| 3 | Steuerung | 971 | zweiter Anschlussstutzen |
| 30 | Verbindungsleitung | 98 | Flächenelement |
| | | 980 | Filmscharnier |
| 4 | Ausgangsleitung | | |

## Patentansprüche

1. Saugpumpeneinheit mit einem Unterdruckanschluss, einem Überdruckanschluss und einem Umschaltventil (2, 9), wobei das Umschaltventil (2, 9) einen Ventilkörper (20, 21, 95) aufweist mit zwei Eingängen (22, 23; 950, 951), wobei ein erster der Eingänge (22, 950) mit dem Unterdruckanschluss und ein zweiter der Eingänge (23, 951) mit dem Überdruckanschluss verbunden ist, und wobei das Umschaltventil (2, 9) einen Ausgang (26, 96) mit einer Durchgangsöffnung aufweist, wobei der Ausgang (26, 96) relativ zu den Eingängen (22, 23; 950, 951) vom ersten Eingang (22, 950) zum zweiten Eingang (23, 951) und zurück bewegbar ist, wodurch die Durchgangsöffnung abwechslungsweise mit einem der Eingänge (22, 23, 950, 951) eine fluidkommunizierende Verbindung bildet, **dadurch gekennzeichnet, dass** die Durchgangsöffnung des Ausgangs (26, 96) während mindestens einem Teil der Bewegung zwischen den zwei Eingängen (22, 23; 950, 951) freigegeben und belüftet ist.

2. Saugpumpeneinheit nach Anspruch 1, wobei der Ausgang (26, 96) beim Bewegen zwischen den zwei Eingängen (22, 23; 950, 951) relativ zu den zwei Eingängen (22,23; 950, 951) anhebbar ist, wobei die Durchgangsöffnung des Ausgangs (26, 96) im angehobenen Zustand freigegeben und belüftet ist.

3. Saugpumpeneinheit nach einem der Ansprüche 1 oder 2, wobei der Ventilkörper (20, 21; 95) in der Saugpumpeneinheit lagefixiert angeordnet ist und der Ausgang (26,96) bewegbar ist.

4. Saugpumpeneinheit nach einem der Ansprüche 1 bis 3, wobei die relative Bewegung des Ausgangs (26, 96) zu den zwei Eingängen (22, 23; 950, 951) eine Kombination einer geradlinigen Verschiebung in einer ersten Richtung und einer Anhebung in einer zur ersten Richtung senkrechten zweiten Richtung ist.

5. Saugpumpeneinheit nach einem der Ansprüche 1 bis 4, wobei am Ausgang (26, 96) eine Ausgangsleitung (4) der Saugpumpeneinheit befestigt ist, welche mit dem Ausgang (26, 96) bewegbar ist.

6. Saugpumpeneinheit nach einem der Ansprüche 1 bis 5, wobei sie eine einzige Vakuumpumpe (1) aufweist, wobei die Vakuumpumpe (1) den Unterdruckanschluss aufweist und wobei sie ferner einen Auspuff aufweist, welche den Überdruckanschluss bildet.

7. Saugpumpeneinheit nach einem der Ansprüche 1 bis 6, wobei die zwei Eingänge (22,23; 950, 951) in einer Ebene nebeneinander angeordnet sind.

8. Saugpumpeneinheit nach einem der Ansprüche 1 bis 7, wobei die zwei Eingängen (22, 23; 950, 951) in einer Gleitplatte (94) mit einer planen Oberfläche angeordnet sind und der Ausgang (26, 96) bei erstellter fluidkommunizierender Verbindung mit den Eingängen (22, 23; 950, 951) auf dieser Gleitplatte (94) aufliegt.

9. Saugpumpeneinheit nach einem der Ansprüche 1 bis 8, wobei das Umschaltventil (2) zwei Elektromagnete mit je einer Spule (20, 21) aufweist, wobei jede Spule (20, 21) von einem hohlen ferromagnetischen Kern (22, 23) durchsetzt ist, wobei die zwei Kerne (22, 23) die zwei Eingänge bilden und wobei der Ausgang einen Permanentmagneten (26) aufweist.

10. Saugpumpeneinheit nach Anspruch 9, wobei der Permanentmagnet ein Ring (26) ist und die Durchgangsöffnung bildet.

11. Saugpumpeneinheit nach einem der Ansprüche 9 oder 10, wobei die Eingänge (22, 23) des Ventilkörpers von einem Deckel (25) überdeckt, welcher über den Eingängen (22, 23) einen Hohlraum (250) freilässt, wobei der Ausgang (26) in diesem Hohlraum (250) bewegbar gehalten ist.

12. Saugpumpeneinheit nach Anspruch 11, wobei der Hohlraum (250) mit Umgebungsluft beaufschlagt ist.

13. Saugpumpeneinheit nach einem der Ansprüche 11 oder 12, wobei der Hohlraum (250) eine Höhe aufweist, welche grösser als eine Dicke des Ausgangs (26) ist.

14. Saugpumpeneinheit nach einem der Ansprüche 9 bis 13, wobei die zwei Spulen (20, 21) in einem gemeinsamen Joch (27) gehalten sind, wobei das Joch (27) an einem dem Ausgang (26) gegenüberliegenden Ende des Ventilkörpers angeordnet ist.

15. Saugpumpeneinheit nach einem der Ansprüche 1 bis 8, wobei das Umschaltventil (2) zwei parallel zueinander angeordnete und parallel zueinander verschiebbare Platten (94, 95) aufweist, wobei in einer ersten dieser Platten (94) der Ausgang (96) gehalten ist und in einer zweiten dieser Platten (95) die zwei Eingänge (950, 951) angeordnet sind.

16. Saugpumpeneinheit nach Anspruch 15, wobei die zwei Platten (94, 95) mittels Biegeelementen, insbesondere Blattfedern (92, 93) oder Flächenelementen (98) mit Filmgelenken (980), miteinander zu einem Parallelogramm verbunden sind.

17. Saugpumpeneinheit nach einem der Ansprüche 15 oder 16, wobei mindestens eine dieser zwei Platten (94, 95) durch Kraftbeaufschlagung in Richtung ihrer Plattenebene bewegbar ist und wobei durch diese Bewegung der Ausgang (96) relativ zu den zwei Eingängen (950, 951) zwischen den zwei Eingängen (950, 951) hin und her bewegbar ist.

18. Verfahren zum Betreiben einer Saugpumpeneinheit gemäss einem der Ansprüche 1 bis 17, wobei die Saugpumpeneinheit einen Unterdruckanschluss, einen Überdruckanschluss und ein Umschaltventil (2, 9) aufweist, wobei das Umschaltventil (2, 9) einen Ventilkörper aufweist mit zwei Eingängen (22, 23; 950, 951), wobei ein erster der Eingänge (22, 950) mit dem Unterdruckanschluss und ein zweiter der Eingänge (23, 951) mit dem Überdruckanschluss verbunden ist, und wobei das Umschaltventil (2, 9) einen Ausgang (26, 96) mit einer Durchgangsöffnung aufweist,
wobei der Ausgang (26, 96) relativ zu den zwei Eingängen (22, 23; 950, 951) zwischen den zwei Eingängen (22, 23; 950,951) hin und her bewegt wird, wobei die Durchgangsöffnung beim Bewegen des Ausgangs (26, 96) abwechslungsweise mit einem der Eingänge (22, 23; 950, 951) eine fluidkommunizierende Verbindung erstellt,
**dadurch gekennzeichnet, dass** die Durchgangsöffnung des Ausgangs (26, 96) beim Bewegen zwischen den zwei Eingängen (22, 23; 950, 951) freigegeben und belüftet wird.

## Claims

1. A suction pump unit with a vacuum port, an excess pressure port and a switching valve (2, 9), wherein the switching valve (2, 9) comprises a valve body (20, 21, 95) with two inputs (22, 23; 950, 951), wherein a first of the inputs (22, 950) is connected with the vacuum port, and a second of the inputs (23, 951) is connected with the excess pressure port, and wherein the switching valve (2, 9) comprises an output (26, 96) with a through hole, wherein the output (26, 96) can be moved relative to the inputs (22, 23; 950, 951) from the first input (22, 950) to the second input (23, 951) and back, as a result of which the through hole alternately establishes a fluid-communicating connection with one of the inputs (22, 23, 950, 951), **characterized in that** the through hole of the output (26, 96) is released and ventilated during at least part of the movement between the two inputs (22, 23; 950, 951).

2. The suction pump unit according to claim 1, wherein the output (26, 96) can be lifted relative to the two inputs (22, 23; 950, 951) while moving between the two inputs (22, 23; 950, 951), wherein the through hole of the output (26, 96) is released and ventilated in the lifted state.

3. The suction pump unit according to one of claims 1 or 2, wherein the valve body (20, 21; 95) is fixed in place in the suction pump unit, and the output (26, 96) can move.

4. The suction pump unit according to one of claims 1 to 3, wherein the movement of the output (26, 96) relative to the two inputs (22, 23; 950, 951) involves a combination of being shifted along a straight line in a first direction and being lifted in a second direction perpendicular to the first direction.

5. The suction pump unit according to one of claims 1 to 4, wherein the output (26, 96) has secured to it an output line (4) of the suction pump unit, which can be moved together with the output (26, 96) .

6. The suction pump unit according to one of claims 1 to 5, wherein it comprises a single vacuum pump (1), wherein the vacuum pump (1) comprises the vacuum port, and wherein it further comprises an exhaust which forms the excess pressure port.

7. The suction pump unit according to one of claims 1 to 6, wherein the two inputs (22, 23; 950, 951) are arranged next to each other in a plane.

8. The suction pump unit according to one of claims 1 to 7, wherein the two inputs (22, 23; 950, 951) are arranged in a sliding plate (94) with a planar surface, and the output (26, 96) rests upon this sliding plate (94) once the fluid-communicating connection with the inputs (22, 23; 950, 951) has been established.

9. The suction pump unit according to one of claims 1 to 8, wherein the switching valve (2) comprises two electromagnets with one coil (20, 21) each, wherein each coil (20, 21) is interspersed by a ferromagnetic, hollow core (22, 23), wherein the two cores (22, 23) form the two inputs, and wherein the output comprises a permanent magnet (26).

10. The suction pump unit according to claim 9, wherein the permanent magnet is a ring (26) and forms the through hole.

11. The suction pump unit according to one of claims 9 or 10, wherein the inputs (22, 23) of the valve body are covered by a lid (25), which opens up a hollow space (25) over the inputs (22, 23), wherein the output (26) is movably held in this hollow space (250).

12. The suction pump unit according to claim 11, wherein the hollow space (250) is subjected to ambient air.

13. The suction pump unit according to one of claims 11 or 12, wherein the hollow space (250) comprises a height that exceeds a thickness of the output (26) .

14. The suction pump unit according to one of claims 9 to 13, wherein the two coils (20, 21) are held in a shared yoke (27), wherein the yoke (27) is arranged at an end of the valve body lying opposite the output (26).

15. The suction pump unit according to one of claims 1 to 8, wherein the switching valve (2) comprises two plates (94, 95) that are arranged parallel to each other and can be shifted parallel to each other, wherein the output (96) is held in a first of these plates (94), and the two inputs (950, 951) are situated in a second of these plates (95) .

16. The suction pump unit according to claim 15, wherein the two plates (94, 95) are connected with each other to form a parallelogram by means of flexural elements, in particular leaf springs (92, 93) or surface elements (98) with film hinges (980) .

17. The suction pump unit according to one of claims 15 or 16, wherein at least one of these two plates (94, 95) can be moved in the direction of its plate plane through the exertion of force, and wherein this motion makes it possible to move the output (96) relative to the two inputs (950, 951), back and forth between the two inputs (950, 951).

18. A method for operating a suction pump unit according to one of claims 1 to 17, wherein the suction pump unit comprises a vacuum port, an excess pressure port and a switching valve (2, 9), wherein the switching valve (2, 9) comprises a valve body (20, 21, 95) with two inputs (22, 23; 950, 951), wherein a first of the inputs (22, 950) is connected with the vacuum port, and a second of the inputs (23, 951) is connected with the excess pressure port, and wherein the switching valve (2, 9) comprises an output (26, 96) with a through hole,
wherein the output (26, 96) is moved relative to the inputs (22, 23; 950, 951), back and forth between two inputs (22, 23; 950, 951),
wherein the through hole alternately establishes a fluid-communicating connection with one of the inputs (22, 23, 950, 951) during the movement of the output (26, 96),
**characterized in that** the through hole of the output (26, 96) is released and ventilated during the movement between the two inputs (22, 23; 950, 951).

## Revendications

1. Unité de pompe aspirante avec un raccord de sous-pression, un raccord de surpression et une soupape d'inversion (2, 9), la soupape d'inversion (2, 9) comportant un corps de soupape (20, 21, 95) doté de deux entrées (22, 23 ; 950, 951), une première des entrées (22, 950) étant reliée au raccord de sous-pression et une deuxième des entrées (23, 951) étant reliée au raccord de surpression et la soupape d'inversion (2, 9) comportant une sortie (26, 96) avec une ouverture traversante, la sortie (26, 96) pouvant être amenée par rapport aux entrées (22, 23 ; 950, 951) de la première entrée (22, 950) à la deuxième entrée (23, 951) et inversement, permettant ainsi à l'ouverture traversante de former alternativement avec une des entrées (22, 23, 950, 951) une liaison en communication fluide, **caractérisée en ce que** l'ouverture traversante de la sortie (26, 96) est libérée et ventilée pendant au moins une partie du mouvement entre les deux entrées (22, 23 ; 950, 951).

2. Unité de pompe aspirante selon la revendication 1, la sortie (26, 96) pouvant être soulevée par rapport aux deux entrées (22, 23 ; 950, 951) lors du déplacement entre les deux entrées (22, 23 ; 950, 951), l'ouverture traversante de la sortie (26, 96) étant libérée et ventilée à l'état soulevé.

3. Unité de pompe aspirante selon l'une quelconque des revendications 1 ou 2, le corps de soupape (20, 21 ; 95) étant disposé de façon à être fixé en place dans l'unité de pompe aspirante et la sortie (26, 96) étant mobile.

4. Unité de pompe aspirante selon l'une quelconque des revendications 1 à 3, le mouvement relatif de la sortie (26, 96) par rapport aux deux entrées (22, 23 ; 950, 951) étant une combinaison d'un coulissement en ligne droite dans une première direction et d'un soulèvement dans une deuxième direction perpendiculaire à la première direction.

5. Unité de pompe aspirante selon l'une quelconque des revendications 1 à 4, une conduite de sortie (4) de l'unité de pompe aspirante étant fixée à la sortie (26, 96), ladite conduite pouvant se déplacer avec la sortie (26, 96).

6. Unité de pompe aspirante selon l'une quelconque des revendications 1 à 5, celle-ci comportant une seule pompe à vide (1), la pompe à vide (1) comportant le raccord de sous-pression et comportant en outre un échappement formant le raccord de surpression.

7. Unité de pompe aspirante selon l'une quelconque des revendications 1 à 6, les deux entrées (22, 23 ; 950, 951) étant disposées côte à côte dans un plan.

8. Unité de pompe aspirante selon l'une quelconque des revendications 1 à 7, les deux entrées (22, 23 ; 950, 951) étant disposées dans une plaque de glissement (94) avec une surface plane et la sortie (26, 96) reposant sur cette plaque de glissement (94) lorsque la liaison en communication fluide est établie avec les entrées (22, 23 ; 950, 951).

9. Unité de pompe aspirante selon l'une quelconque des revendications 1 à 8, la soupape d'inversion (2) comportant deux électroaimants avec respectivement une bobine (20, 21), chaque bobine (20, 21) étant traversée d'un noyau ferromagnétique creux (22, 23), les deux noyaux (22, 23) formant les deux entrées et la sortie comportant un aimant permanent (26).

10. Unité de pompe aspirante selon la revendication 9, l'aimant permanent étant un anneau (26) et formant l'ouverture traversante.

11. Unité de pompe aspirante selon l'une quelconque des revendications 9 ou 10, les entrées (22, 23) du corps de soupape étant recouvertes d'un cache (25) libérant au-dessus des entrées (22, 23) un espace creux (250), la sortie (26) étant maintenue de façon mobile dans cet espace creux (250).

12. Unité de pompe aspirante selon la revendication 11, l'espace creux (250) étant alimenté en air ambiant.

13. Unité de pompe aspirante selon l'une quelconque des revendications 11 ou 12, l'espace creux (250) présentant une hauteur supérieure à une épaisseur de la sortie (26).

14. Unité de pompe aspirante selon l'une quelconque des revendications 9 à 13, les deux bobines (20, 21) étant maintenues dans un joug (27) commun, le joug (27) étant disposé au niveau d'une extrémité du corps de soupape opposée à la sortie (26).

15. Unité de pompe aspirante selon l'une quelconque des revendications 1 à 8, la soupape d'inversion (2) comportant deux plaques (94, 95) disposées parallèlement l'une par rapport à l'autre et pouvant être coulissées parallèlement l'une par rapport à l'autre, sachant que la sortie (96) est maintenue dans une première de ces plaques (94) et que les deux entrées (950, 951) sont disposées dans une deuxième de ces plaques (95).

16. Unité de pompe aspirante selon la revendication 15, les deux plaques (94, 95) étant reliées en elles en parallélogramme à l'aide d'éléments de flexion, notamment de ressorts à lames (92, 93) ou d'éléments de surface (98) dotés d'articulations filmées (980).

17. Unité de pompe aspirante selon l'une quelconque des revendications 15 ou 16, au moins une de ces deux plaques (94, 95) pouvant être déplacée par application de force en direction de leur plan de plaque et ce mouvement permettant à la sortie (96) d'effectuer un mouvement de va-et-vient par rapport aux deux entrées (950, 951), entre les deux entrées (950, 951).

18. Procédé d'entraînement d'une unité de pompe aspirante selon l'une quelconque des revendications 1 à 17, l'unité de pompe aspirante comportant un raccord de sous-pression, un raccord de surpression et une soupape d'inversion (2, 9), la soupape d'inversion (2, 9) comportant un corps de soupape doté de deux entrées (22, 23 ; 950, 951), une première des entrées (22, 950) étant reliée au raccord de sous-pression et une deuxième des entrées (23, 951) étant reliée au raccord de surpression et la soupape d'inversion (2, 9) comportant une sortie (26, 96) dotée d'une ouverture traversante ;
la sortie (26, 96) effectuant un mouvement de va-et-vient par rapport aux deux entrées (22, 23 ; 950, 951), entre les deux entrées (22, 23 ; 950, 951) ; l'ouverture traversante établissant, alternativement avec une des entrées (22, 23 ; 950, 951), une liaison en communication fluide lors du déplacement de la sortie (26, 96) ;
**caractérisé en ce que** l'ouverture traversante de la sortie (26, 96) est libérée et ventilée lors du déplacement entre les deux entrées (22, 23 ; 950, 951).
